# EUROPEAN PATENT APPLICATION

(11) **EP 1 388 333 A1**
(43) Date of publication of application: **11.02.2004**
(21) Application number: 03007533.7
(22) Date of filing: 01.04.2003
(51) Int. Cl.: A61F 13/00, A61F 5/37

(54) **Medical weight**

(30) Priority: 09.08.2002 JP 2002232617
(71) Applicant: Colin Corporation, Komaki-shi, Aichi-ken (JP)
(72) Inventor: Ohama, Komei, Komaki-shi, Aichi-ken (JP)
(74) Representative: Winter, Brandl, Fürniss, Hübner, Röss, Kaiser, Polte Partnerschaft

(57) **Abstract**

A medical weight (10) for being placed on a sensor (28) worn on a body surface (26) of a living subject, so as to press the sensor against the body surface, the medical weight including one or more bags (18,22), and a number of spheroidal bodies (24) accommodated in the bag or bags.

## Description

### BACKGROUND OF THE INVENTION

### Field of the Invention

The present invention relates to a medical weight for being used in obtaining physical information from a living being by, e.g., pressing a clinical sensor on the living being.

### Object of the Invention

An object of the present invention is to provide a medical weight which enjoys at least one of the following advantages: it has a simple structure; it applies equal weights; it presses an object in a well-balanced or stable state; it more or less holds an object; it has a shape that can be reduced in size and/or thickness; it exhibits high sound-isolating and/or shielding effects; and it improves signal/noise ratio (i.e., S/N ratio).

### Related Art Statement

The present invention is applicable to, e.g., a measurement using a heart-sound sensor, as follows: The heart-sound sensor is worn on the chest of a living being so as to obtain a phonocardiogram and make a diagnosis on a disease such as heart disease or respiratory-system disease. Generally, the photocardiogram is measured in a calm room, such as an anacoustic room, so as to prevent its mixture with external noise such as person's talking voices or walking sounds, or door's opening and closing sounds, and thereby improve its S/N ratio. In addition, if the heart-sound sensor is held in close contact with the body surface of the living subject, then the S/N ratio is improved. Thus, a medical weight is known which is placed on the heart-sound sensor worn on the body surface of the subject so as to press the sensor against the body surface. Generally, the conventional medical weight consists of a sand bag that includes a bag and sand accommodated in the bag.

However, the conventional medical weight consisting of the sand bag has the following problems:
(1) In order to keep the heart-sound sensor in close contact with the living subject, an appropriate weight distribution needs to be kept for a certain time period. However, it is difficult to equally distribute the sand in the sand bag.
(2) Since the sand is not equally distributed in the sand bag, it is difficult to press, with the sand bag, the heart-sound sensor in a stable state.
(3) In addition, the sand bag cannot fit or hold the subject.
(4) Accordingly, the heart-sound sensor is easily influenced by foreign electromagnetic waves.
(5) The sand bag cannot sufficiently prevent foreign noise from mixing with the signal detected by the heart-sound sensor.
(6) In order to provide the sand bag with an appropriate weight by increasing the amount of sand accommodated in the bag, it is needed to increase the thickness or size of the sand bag, and thereby increase the dimension of the medical weight.

### SUMMARY OF THE INVENTION

The present invention has been developed to solve at least one of the above-indicated problems.

According to the present invention, there is provided a medical weight for being placed on a sensor worn on a body surface of a living subject, so as to press the sensor against the body surface.
(1) The medical weight comprises at least one bag; and a number of spheroidal bodies which are accommodated in the at least one bag. The number of spheroidal bodies may comprise a number of small spheroidal weights. According to this feature, since the small weights are spheroidal, the area of contact of each small weight with other small weights is reduced and accordingly each small weight can easily move. Therefore, in the state in which the medical weight presses the sensor against the body surface, the medical weight can easily deform according to the shape of the sensor and/or the body surface underlying it.
(2) In addition, since the small weights accommodated in the bag are spheroidal, spaces are formed among the small weights in the bag. Those spaces can prevent foreign noise from being transmitted to the sensor via the medical weight.
(3) Each of the spheroidal bodies may consist of a true sphere. The spheroidal bodies may comprise small spheroidal weights. According to this feature, since each small weight contacts, at points, with other small weights, each small weight can most easily move, and the largest spaces are formed among the small weights. Therefore, the medical weight can press the sensor in a stable state, and can more effectively prevent the transmission of the foreign noise to the sensor.
(4) The true spheres may have a diameter of from 0.5 mm to 2.0 mm. According to this feature, the medical weight can most comfortably fit or hold the subject.
(5) The medical weight may comprise a main bag which is adapted to be placed on the sensor, and at least two side bags which are provided on two sides of, or around, the main bag. According to this feature, the small weights accommodated in the main bag are equally distributed so that the small weights can equally press the sensor against the body. Thus, the medical weight can press the sensor in a stable state.
(6) In addition, a central portion of the main bag presses the sensor, and the side bags hang onto the body surface so as to closely contact the subject. Thus, the medical weight can press the sensor in a more stable state.
(7) In addition, since the medical weight isolates the sensor from outside, the foreign noise is prevented and accordingly the SN ratio is improved.
(8) In addition, each of the spheroidal bodies is formed of a heavy metal. The heavy metal may be gold, platinum, silver, copper, lead, iron, or other metals having a specific gravity of not small than 4. According to this feature, the medical weight can be given an appropriate weight, without having to increase any dimensions thereof. Thus, the medical weight can be formed in a small size and/or with a small thickness.
(9) When the small weights are formed of a heavy metal having a greater specific gravity, the medical weight exhibits a higher sound-isolating effect. In particular, since a soft metal such as copper, lead, or their alloy exhibits a high vibration-absorbing effect, the medical weight including the small weights formed of the soft metal, exhibits a significantly high isolating effect against foreign noise.
(10) In addition, in the case where the small weights are formed of the heavy metal, the sensor is surrounded by the heavy metal. Thus, the sensor is isolated from foreign electromagnetic waves and accordingly the S/N ratio is further improved.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a plan view of a medical weight to which the present invention is applied;
Fig. 2 is a cross-section view taken along 2 - 2 in Fig. 1; and
Fig. 3 is a view showing a state in which the medical weight is used.

### DETAILED DESCRIPTION OF PREFERRED EMBODIMENTS

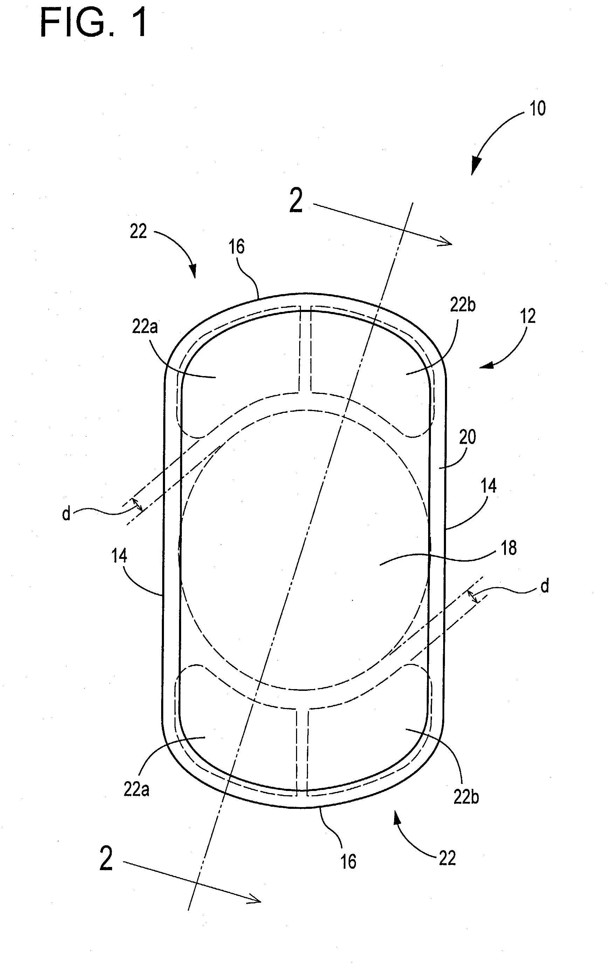

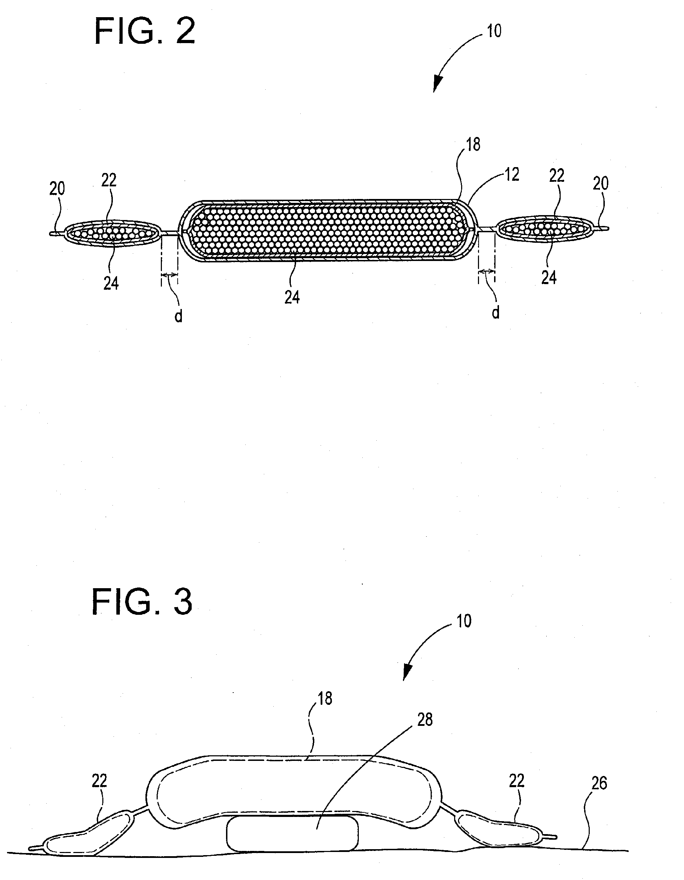

Hereinafter, there will be described an embodiment of the present invention in detail by reference to the drawings. Fig. 1 is a plan view of a medical weight 10 to which the present invention is applied; and Fig. 2 is a cross-section view taken along 2 - 2 in Fig. 1.

As shown in Fig. 1, the medical weight 10 has, in the plan view, a generally rectangular shape whose corners are rounded. As shown in Figs. 1 and 2, the medical weight 10 includes a flexible, outer cover 12 that wholly covers the device 10. The plan-view shape of the medical weight 10, i.e., the plan-view shape of the outer cover 12 has a pair of straight sides 14 that are substantially parallel to each other, and a pair of arcuate sides 16 that connect between opposite ends of one of the two straight sides 14 and opposite ends of the other straight side 14. The size of the medical weight 10 is not limited all. However, in the case where the medical weight 10 is used by being placed on a heart-sound sensor, it is preferred that the medical weight 10 be larger than the heart-sound sensor. For example, the medical weight 10 has a length of 20 cm (in a vertical direction in Fig. 1) and a width of 12 cm (in a horizontal direction in Fig. 1).

The outer cover 12 accommodates, in a generally central portion thereof, a large bag (i.e., a main bag) 18 that has a generally circular shape in the plan view. The large bag 18 is formed of a flexible material such as a synthetic fiber. The large bag 18 has, in the widthwise direction of the outer cover 12, a dimension that is somewhat smaller than the width of the outer cover 12, and an edge portion 20 having a width of about 5 mm partly surrounds the large bag 18 and defines the two straight sides14 of the outer cover 12. The large bag 18 has, in the lengthwise direction of the outer cover 12, a dimension that assures that two small bags (i.e., two side bags) 22 are respectively accommodated between the two arcuate sides 16 of the outer cover 12, and the large cover 18. The edge portion 20 defines the entire periphery of the outer cover 12.

Each of the two small bags 22 is formed like a belt from one of the two straight sides 14 to the other straight side 14, along a corresponding one of the two arcuate sides 16 of the outer cover 12, between the one arcuate side 16 and the large bag 18. Each small bag 22 is divided, at a widthwise middle portion of the outer cover 12, into two portions, i.e., a first small bag 22a and a second small bag 22b that are line-symmetric with each other. The small bags 22 are also formed of a flexible material such as a synthetic fiber.

The large bag 18 and each of the small bags 22 are distant from each other by a distance, d, and, in this state, the large and small bags 18, 22 are fixed, by sewing, to the outer cover 12.

As shown in Fig. 2, each of the large and small bags 18, 22 accommodates a number of small weights 24 each of which is a true sphere. The small weights 24 are formed of steel, and each small weight is sufficiently smaller than the large bag 18 or each small bag 22 and has, for example, a diameter of from 0.5 mm to 2.0 mm. The large bag 18 accommodates small weights 24 that weigh, e.g., 550 g; and each of the first and second small bags 22a, 22b accommodates small weights 24 that weigh, e.g., 30 g.

Fig. 3 shows a state in which the medical weight 10 is used. In Fig. 3, a heart-sound sensor 28 is placed at a prescribed position on a body surface 26 of the chest of a living subject, and the medical weight 10 is placed on the heart-sound sensor 28 such that the sensor 28 underlies a central portion of the large bag 18. The heart-sound sensor 28 may be of any type such as air-conduction type, direct-conduction type (acceleration type, placement type), etc.

As shown in Fig. 3 in which the medical weight 10 is placed on the heart-sound sensor 28, an outer peripheral portion of the large bag 18 that does not ride on the sensor 28 hangs over the body surface 26. In addition, the small bags 22 hang down onto the body surface 26 and contact the same 26. Thus, the medical weight 10 can press the sensor 28 in a stable state.

In the illustrated embodiment, the small weights 24 are formed of steel that has a great specific gravity. Thus, the medical weight 10 can be given an appropriate weight, without having to increase any dimensions of the same 10. In addition, the greater the specific gravity is, the higher sound-isolating effect the small weights 24 exhibit. Thus, the medical weight 10 can effectively prevent the transmission of foreign noise to the heart-sound sensor 28 via the same 10.

In addition, in the illustrated embodiment, the small weights 24 have a spheroidal shape. Thus, the area of contact of each small weight 24 with other small weights 24 is reduced, and accordingly each small weight 24 can easily move. Therefore, in the state in which the medical weight 10 presses the heart-sound sensor 28, the weight device 10 deforms according to the shape of the sensor 28 and/or the body surface 26 underlying the same 10. Thus, the medical weight 10 can press the sensor 28 in a stable state. In addition, since the small weights 24 accommodated in the large and small bags 18, 22 have the spheroidal shape, spaces are formed in the bags 18, 22 and accordingly the medical weight 10 can more effectively prevent the transmission of foreign noise to the heart-sound sensor 28 via the same 10.

In the illustrated embodiment, since each small weight 24 consists of a true sphere, each small weight 24 contacts, at points, with other small weights 24. Thus, the small weights 24 can most easily move, and the largest spaces are formed in the large and small bags 18, 22. Therefore, the medical weight 10 can press the sensor 28 in a more stable state, and can more effectively isolate the foreign noise.

Moreover, in the illustrated embodiment, the medical weight 10 includes the large bag 18 that is adapted to be placed on the heart-sound sensor 28, and the small bags 22 that are provided on the two sides of the large bag 18, respectively. Thus, in the state in which the large bag 18 that is placed on the heart-sound sensor 28, the small bags 22 hang onto the body surface 26. Therefore, the medical weight 10 can press the sensor 28 in a still more stable state.

While the present invention has been described in its embodiment in detail by reference to the drawings, it is to be understood that the present invention can otherwise be embodied.

For example, in the illustrated embodiment, the small weights 24 are formed of steel. However, the small weights 24 may be formed of a different sort of heavy metal such as copper or lead or an alloy of heavy metals.

In addition, in the illustrated embodiment, each of the small weights 24 consists of a true sphere. However, each small weight 24 may have any shape such as a spheroidal shape other than the true sphere, or a cubic shape.

In addition, the illustrated medical weight 10 includes the two small bags 22 on the two sides of the large bag 18, respectively. However, the small bags 22 may be omitted.

In addition, in the illustrated embodiment, the medical weight 10 is used with the heart-sound sensor 28. However, the medical weight 10 may be used with a different sort of medical sensor that is used by being worn on a body surface of a living subject; such as a body-temperature sensor or a probe that is employed in a reflection-type oximeter. In the case where the medical weight 10 is used with the probe employed in the reflection-type oximeter, the weight 10 can exhibit a light-shielding effect, i.e., prevents a light-receiving element of the probe from receiving foreign lights other than a light emitted by a light-emitting element of the probe.

It emerges from the foregoing description that the medical weight according to the present invention can enjoy the following advantages:
(1) The medical weight easily applies equal weights to an object.
(2) The medical weight presses an object in a well-balanced or stable manner, and more or less holds the object.
(3) The medical weight has a shape that can be reduced in size and thickness.
(4) The medical weight exhibits a sound-isolating effect and a shielding effect, and improves a signal/noise ratio (i.e., an S/N ratio).

While the present invention has been described in detail in its embodiments by reference to the drawings, it is to be understood that the present invention is not limited to the details of the described embodiments but may be embodied with various changes or improvements that may occur to a person skilled in the art.

## Claims

1. A medical weight (10) for being placed on a sensor (28) worn on a body surface (26) of a living subject, so as to press the sensor against the body surface, the medical weight comprising:
at least one bag (18, 22); and
a number of spheroidal bodies (24) which are accommodated in said at least one bag.

2. A medical weight according to claim 1, wherein each of the spheroidal bodies (24) consists of a true sphere.

3. A medical weight according to claim 2, wherein the true spheres (24) have a diameter of from 0.5 mm to 2.0 mm.

4. A medical weight according to any of claims 1 through 3, comprising a main bag (18) which is adapted to be placed on the sensor (28), and at least two side bags (22) including at least one first side bag (22a, 22b) which is provided on one of two sides of the main bag and at least one second bag (22a, 22b) which is provided on the other side of the main bag.

5. A medical weight according to claim 4, comprising at least four side bags (22a, 22b) including at least two first side bags (22a, 22b) which are provided on said one side of the main bag (18) and at least two second side bags (22a, 22b) which are provided on said other side of the main bag.

6. A medical weight according to any of claims 1 through 5, wherein each of the spheroidal bodies (24) is formed of a heavy metal.

7. A medical weight according to any of claims 1 through 5, wherein each of the spheroidal bodies (24) is formed of copper.

8. A medical weight according to any of claims 1 through 5, wherein each of the spheroidal bodies (24) is formed of steel.

9. A medical weight according to any of claims 1 through 5, wherein each of the spheroidal bodies (24) is formed of lead.
